# EUROPÄISCHE PATENTSCHRIFT

(11) **EP 2 668 151 B1**
(45) Veröffentlichungstag und Bekanntmachung des Hinweises auf die Patenterteilung: **16.11.2016**
(21) Anmeldenummer: 12700495.0
(22) Anmeldetag: 20.01.2012
(51) Int. Cl.: C07C 209/08, C07C 209/62, C07C 211/15, C07D 207/40

(54) **VERFAHREN ZUR HERSTELLUNG VON 2,2-DIFLUORETHYLAMIN AUSGEHEND VON 2,2-DIFLUOR-1-CHLORETHAN**
METHOD FOR PRODUCING 2,2-DIFLUOROETHYLAMINE BASED ON 2,2-DILFUORO-1-CHLOROETHANE
PROCÉDÉ DE FABRICATION DE 2,2-DIFLUOROÉTHYLAMINE À PARTIR DE 2,2-DIFLUORO-1-CHLORÉTHANE

(30) Priorität: 24.01.2011 EP 11151873; 24.01.2011 US 201161435497 P
(43) Veröffentlichungstag der Anmeldung: 04.12.2013
(73) Patentinhaber: Bayer Intellectual Property GmbH, 40789 Monheim am Rhein (DE)
(72) Erfinder: LUI, Norbert, 51519 Odenthal (DE); HEINRICH, Jens-Dietmar, 51368 Leverkusen (DE)
(74) Vertreter: BIP Patents
(86) Internationale Anmeldenummer: PCT/EP2012/050834
(87) Internationale Veröffentlichungsnummer: WO 2012/101044

(56) Entgegenhaltungen:
- WO-A1-2009/036901
- OBWOHLM HUDLICKY: "Dehydrohalogenation", CHEMISTRY OF ORGANOFLUORINE COMPOUNDS, 1976, Seiten 489-495, XP009146465,
- GUIDO VERNIEST ET AL: "Synthesis and Reactivity of 1-Substituted 2-Fluoro- and 2,2-Difluoroaziridines", J. ORG. CHEM., Bd. 72, Nr. 22, 2007, Seiten 8569-8572, XP002630131,
- DATABASE WPI Week 200025 Thomson Scientific, London, GB; AN 2000-285445 XP002630971, & HU 9 702 472 A2 (RICHTER GEDEON VEGYESZET) 28. Februar 2000 (2000-02-28)
- EVANS, R. ET AL: "2-Fluoro- and 2,2-Difluoroethylnitroguanidine", JOURNAL OFORGANIC CHEMISTRY, Bd. 23, 1958, Seiten 1077-1078, XP002630972,

## Beschreibung

Die vorliegende Erfindung betrifft ein Verfahren zur Herstellung von 2,2-Difluorethylamin ausgehend von 2,2-Difluor-1-chlorethan.

2,2-Difluorethylamin ist eine wichtige Zwischenverbindung bei der Wirkstoffherstellung. Es sind verschiedene Herstellungsmethoden für 2,2-Difluorethylamin bekannt (z.B. Swarts et al. Chem. Zentralblatt, Band 75, 1904, Seiten 944-945; Dickey et al. Industrial and Engineering Chemistry 1956, Nr. 2, 209-213). Die bekannten Verfahren sind nachteilig, da sie entweder eine sehr lange Reaktionszeit bei nur geringer Ausbeute haben oder weil die Reaktionsmischungen stark korrodierend sind, weshalb sich die bekannten Verfahren nicht zum großtechnischen Einsatz eignen.

Ausgehend von den bekannten Verfahren zur Herstellung von 2,2-Difluorethylamin stellt sich nun die Aufgabe, wie 2,2-Difluorethylamin einfach und kostengünstig hergestellt werden kann. Die Erfinder haben gefunden, dass sich 2,2-Difluorethylamin besonders vorteilhaft herstellen lässt, wenn man zuerst eine Imid-Zwischenstufe herstellt und diese dann spaltet.

Gegenstand der Erfindung ist deshalb ein Verfahren zur Herstellung von 2,2-Difluorethylamin, das folgende Schritte umfasst:
Schritt (i): Umsetzen von 2,2-Difluor-1-chlorethan der Formel (I)

   CHF₂-CH₂Cl (I),

   mit einem Imid der Formel (II) 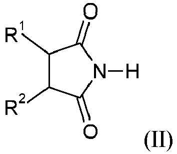 in Gegenwart einer Base, zu einer Verbindung der Formel (III) 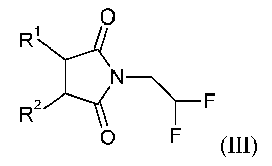 worin in den Verbindungen der Formeln (II) und (III), R¹ und R² jeweils unabhängig voneinander für Wasserstoff oder C₁-C₆-Alkyl stehen, oder R¹ und R² zusammen mit den Kohlenstoffatomen an die sie gebunden sind einen sechsgliedrigen aromatischen Ring bilden, der gegebenenfalls substituiert ist; vorzugsweise ist der sechsgliedrige Ring ggf. mit Halogen oder C₁-C₁₂-Alkyl substituiert; und worin das molare Verhältnis von Base zum eingesetzten Imid der Formel (III) im Bereich von 0,8 bis 5 liegt;
Schritt (ii): Abspaltung von 2,2-Difluorethylamin durch Reaktion der Verbindung der Formel (III) mit Säure, Base oder Hydrazin.

Das in Schritt (i) eingesetzte Imid der Formel (II) kann auch als Salz vorliegen. Solche Salze sind zum Teil käuflich erhältlich (z.B. Kaliumsalz von Phthalimid). Bevor das Salz im erfindungsgemäßen Verfahren eingesetzt wird kann das Imid der Formel (II) auch durch Umsetzung mit einer geeigneten Base zu einem Salz überführt werden. Geeignete Basen sind dem Fachmann bekannt bzw. umfassen die vorliegend genannten Basen.

Im erfindungsgemäßen Verfahren ist es bevorzugt, eine Verbindung der Formel (II) einzusetzen, in der R¹ und R² jeweils für Wasserstoff stehen (d.h. Succinimid), oder in der R¹ und R² zusammen mit den Kohlenstoffatomen an die sie gebunden sind einen sechsgliedrigen aromatischen Ring bilden (d.h. Phthalimid). Wird Succinimid als Verbindung der Formel (II) eingesetzt, so wird im Schritt (i) die Verbindung der Formel (III-a) erhalten, die neu ist. Wird Phthalimid als Verbindung der Formel (II) eingesetzt, so wird im Schritt (i) die Verbindung der Formel (III-b) erhalten:

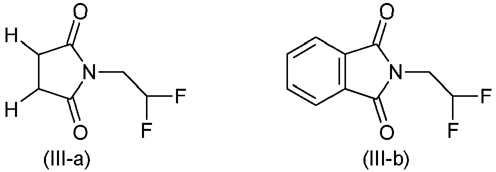

Das erfindungsgemäße Verfahren kann durch das folgende Schema veranschaulicht werden:

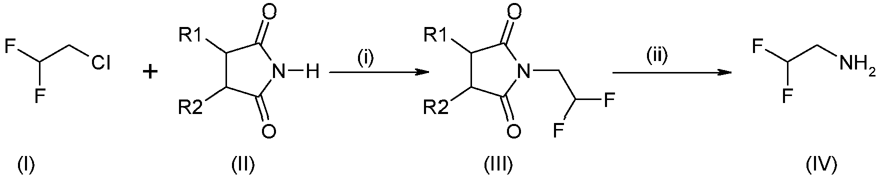

Obwohl aus Chemistry of Organofluorine Compounds (1976), 2. Auflage, S. 489-490 und Houben Weyl, E 10b/2, S. 92-98 bekannt ist, dass eine 2,2-Difluor-1-halogenethan-Verbindung unter basischen Bedingungen unter Abspaltung von HCl, HBr oder HJ zu Vinylidenfluorid reagiert und es dadurch für die Umsetzung in Schritt (i) nicht mehr zur Verfügung steht, und obwohl aus J. Org. Chem. 2007, 72 (22) S. 8569 bekannt ist, dass 2,2-Difluorethylamin sehr reaktiv ist und es deshalb sehr wahrscheinlich ist, dass das erhaltene Imid der Formel (III) unter den erfindungsgemäßen Reaktionsbedingungen des Schrittes (i) weiterreagiert, haben die Erfinder überraschenderweise gefunden, dass das Imid der Formel (III) in guter Ausbeute und Reinheit erhalten wird. Eine umfangreiche Aufreinigung kann deshalb entfallen. Letztendlich wird deshalb auch die Zielverbindung 2,2-Difluorethylamin in einer sehr guten Ausbeute, bezogen auf die im Schritt (i) eingesetzten Edukte, erhalten.

Gleichfalls war es überraschend, dass das in Schritt (i) verwendete 2,2-Difluor-1-chlorethan sich sehr gut und mit einer Ausbeute von über 90 % zum Imid der Formel (III) umsetzen lässt. Es ist nämlich bekannt, dass Chloralkane nicht so reaktiv sind wie Brom- oder Jodalkane und deshalb nicht sehr effizient mit Imiden, insbesondere substituierten oder unsubstituierten Phthalimiden, reagieren.

Die Verwendung von 2,2-Difluor-1-bromethan zur Herstellung von N-(2,2-Difluorethyl)-phthalimid (entspricht der Verbindung der Formel (III-b)) ist bekannt und von Evans, R., Milani, V., Hafner, L. S., Skolnik, Sol. in Journal of Organic Chemistry (1958), 23, S. 1077-1078 beschrieben. In der beschriebenen Reaktion wird 2,2-Difluor-1-bromethan mit Kaliumphthalimid in DMF bei 210°C umgesetzt, wobei N-(2,2-Difluorethyl)-phthalimid in einer Ausbeute von 47 % erhalten wird.

Die von Evans et al. beschriebene Methode zur Herstellung von N-(2,2-Difluorethyl)-phthalimid ist nachteilig, da zum einen bei sehr hohen Temperaturen gearbeitet werden muss und zum anderen die Ausbeute nur 47 % beträgt. Gleichfalls hat das Verfahren eine ungünstige Materialbilanz, da bei der Reaktion ca. 50 % der Masse an eingesetztem 2,2-Difluor-1-bromethan aufgrund des hohen Molekulargewichts von Brom verloren geht.

Unter Materialbilanz wird im Allgemeinen die nach Arten geordnete Gegenüberstellung der Mengen an Materialeinsatz (Input) und Materialausbringung (Output) eines produktiven Systems verstanden. Bei guter Materialbilanz entspricht die Menge (Masse) des Inputs der Menge (Masse) des Outputs.

Das Verwenden von 2,2-Difluor-1-chlorethan in der erfindungsgemäßen Reaktion trägt dazu bei, dass die Reaktion eine bessere Materialbilanz hat.

Verbindungen der Formel (II) sind bekannt, käuflich erhältlich oder können nach üblichen Methoden hergestellt werden.

Sofern nicht anders angegeben, bezieht sich der Ausdruck "Alkyl" in Alleinstellung oder in Kombination mit anderen Begriffen, wie z.B. im Zusammenhang mit den erfindungsgemäßen Katalysatoren erwähnt, beispielsweise Tetraalkylammoniumbromide, Tetraalkylammoniumiodide, Tetraalkylphosphoniumhalogenide, auf lineare oder verzweigte gesättigte Kohlenwasserstoffketten mit bis zu 12 Kohlenstoffatomen, d.h. C₁-C₁₂-Alkyl, bevorzugt mit bis zu 6 Kohlenstoffen, d.h. C₁-C₆-Alkyl, ganz bevorzugt mit bis zu 4 Kohlenstoffen, d.h. C₁-C₄-Alkyl. Beispiele solcher Alkyle sind Methyl, Ethyl, n-Propyl oder Isopropyl, n-, i-, s- oder t-Butyl, n-Pentyl oder n-Hexyl, n-Heptyl, n-Octyl, n-Nonyl, n-Decyl, n-Undecyl und n-Dodecyl. Die Alkyle können mit geeigneten Substituenten substituiert sein, z.B. mit Halogen.

Sofern nichts anderes angegeben, bezieht sich der Ausdruck "Aryl" oder "sechsgliedriger aromatischer Ring" auf einen Phenylring.

Sofern nichts anderes angegeben, steht "Halogen" oder "Hal" für Fluor, Chlor, Brom oder Jod.

Die Umsetzung von 2,2-Difluor-1-chlorethan der Formel (I) mit einem Imid der Formel (II) in Schritt (i) kann in Substanz, d.h. ohne Hinzufügen eines Lösungsmittels oder in Gegenwart eines Lösungsmittels durchgeführt werden.

Im Fall, dass in Schritt (i) ein Lösungsmittel zum Reaktionsgemisch hinzugefügt wird, wird es vorzugsweise in einer solchen Menge eingesetzt, dass das Reaktionsgemisch während des ganzen Verfahrens gut rührbar bleibt. Vorteilhafterweise wird, bezogen auf das Volumen des eingesetzten 2,2-Difluor-1-chlorethan, die 1- bis 50-fache Lösungsmittelmenge, bevorzugt die 2- bis 40-fache Lösungsmittelmenge, besonders bevorzugt die 2- bis 20-fache Lösungsmittelmenge verwendet. Unter Lösungsmittel werden erfindungsgemäß auch Gemische reiner Lösungsmittel verstanden.

Geeignete Lösungsmittel sind alle unter den Reaktionsbedingungen inerten organischen Lösungsmittel. Erfindungsgemäß geeignete Lösungsmittel sind insbesondere Ether (z.B. Ethylpropylether, Methyl-tert-butylether, *n*-Butylether, Anisol, Phenetol, Cyclohexylmethylether, Dimethylether, Diethylether, Dimethylglycol, Diphenylether, Dipropylether, Diisopropylether, Di-*n*-butylether, Diisobutylether, Diisoamylether, Ethylenglycoldimethylether, Isopropylethylether, Diethylenglykoldimethylether, Triethylenglykoldimethylether, Tetrahydrofuran, 2-Methyltetrahydrofuran, Dioxan, und Polyether des Ethylenoxids und/oder Propylenoxids); Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Tetramethylensulfon; aliphatische, cycloaliphatische oder aromatische Kohlenwasserstoffe (z.B. Pentan, Hexan, Heptan, Oktan, Nonan wie die sogenannten "White Spirits" mit Komponenten mit Siedepunkten im Bereich beispielsweise von 40°C bis 250°C, Cymol, Benzinfraktionen innerhalb eines Siedeintervalls von 70°C bis 190°C, Cyclohexan, Methylcyclohexan, Petrolether, Ligroin, Oktan, Benzol, Toluol, Xylol); halogenierte Aromaten (z.B. Chlorbenzol, Dichlorbenzol); Amide (z.B. Hexamethylphosphorsäuretriamid, Formamid, N,N-Dimethyl-acetamid, *N*-Methyl-formamid, *N,N*-Dimethylformamid, *N,N*-Dipropyl-formamid, *N,N*-Dibutyl-formamid, *N*-Methyl-pyrrolidon, *N*-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, *N-*Formyl-piperidin, *N,N*'-1,4-Diformyl-piperazin); Nitrile (z.B. Acetonitril, Propionitril, n- oder iso-Butyronitril oder Benzonitril); Ketone (z.B. Aceton) oder Gemische davon.

Bevorzugte Lösungsmittel in Schritt (i) sind N,N-Dimethylformamid, N,N-Dimethyl-acetamid, Tetramethylensulfon und *N*-Methyl-pyrrolidon.

Im Schritt (i) kann gegebenenfalls auch ein Katalysator vorhanden sein/zugegeben werden. Zur Verwendung im erfindungsgemäßen Verfahren sind alle Katalysatoren geeignet, die die Umsetzung mit dem 2,2-Difluor-1-chlorethan beschleunigen. Mischungen geeigneter Katalysatoren sind auch denkbar. Erfindungsgemäß geeignet sind insbesondere Alkalibromide und -jodide (z.B. Natriumjodid, Kaliumjodid, Kaliumbromid); Ammoniumbromid und Ammoniumjodid; Tetraalkylammoniumbromide und -jodide (z.B. Tetraethylammoniumjodid, Tetrabutylammoniumbromid); bestimmte Phosphoniumhalogenide, wie Tetraalkyl- oder Tetraarylphosphoniumhalogenide (z.B. Hexa-decyl-tri-butyl-phosphoniumbromid, Stearyltributylphosphoniumbromid, Tetrabutylphosphoniumbromid, Tetraoctylphosphoniumbromid, Tetraphenylphosphoniumchlorid und Tetraphenylphosphoniumbromid), Tetrakis(dimethylamino)phosphoniumbromid, Tetrakis(diethylamino)phosphoniumbromid, Tetrakis(dipropylamino)phosphoniumchlorid bzw. -bromid; sowie Bis(dimethylamino)[(1,3-dimethylimidazolidin-2-yliden)amino]methyliumbromid.

Im erfindungsgemäßen Verfahren werden als Katalysatoren bevorzugt Natriumbromid, Kaliumbromid, Natriumjodid, Kaliumjodid, Tetrabutylammoniumbromid oder Tetraphenylphosponiumbromid, besonders bevorzugt Tetrabutylammoniumbromid, insbesondere Tetra-n-butylammoniumbromid, Natrium- oder Kaliumjodid eingesetzt.

Der Katalysator kann auch in-situ erzeugt werden, beispielsweise durch eine Reaktion von HBr oder HJ mit Ammoniak. Weiterhin kann der Katalysator auch *in-situ* durch Zugabe von sehr reaktiven Alkylbromiden oder -jodiden (z.B. Methyl- oder Ethylbromid oder -jodid) erzeugt werden.

Im erfindungsgemäßen Verfahren wird der Katalysator, bezogen auf das eingesetzte Imid der Formel (II), in einer Konzentration von etwa 0,01 bis etwa 25 Gew.-% verwendet. Höhere Konzentrationen sind grundsätzlich möglich. Bevorzugt wird der Katalysator in einer Konzentration von etwa 0,2 bis etwa 25 Gew.-%, besonders bevorzugt von etwa 0,4 bis etwa 20 Gew.-%, ganz besonders bevorzugt von etwa 0,5 bis etwa 15 Gew.-%. verwendet. Der Katalysator kann aber auch bevorzugt in einer Konzentration von etwa 0,05 bis etwa 4 Gew.-%, von etwa 0,1 bis etwa 11 Gew.-% oder von etwa 0,5 bis etwa 11 Gew.-% eingesetzt werden.

Die Umsetzung des Schritts (i) erfolgt vorteilhafterweise in Gegenwart eines oder mehrerer Säurefänger, die in der Lage sind, den bei der Reaktion freiwerdenden Chlorwasserstoff zu binden, hierdurch wird die Ausbeute gesteigert.

Geeignete Säurefänger sind organische und anorganische Basen, die in der Lage sind, den frei werdenden Chlorwasserstoff zu binden. Beispiele für organische Basen sind tertiäre Stickstoffbasen, wie z.B. tertiäre Amine, substituierte oder unsubstituierte Pyridine und substituierte oder unsubstituierte Chinoline, Triethylamin, Trimethylamin, Diisopropylethylamin, Tri-n-propylamin, Tri-n-butylamin, Tri-n-hexylamin, Tricyclohexylamin, N-Methyl-cyclohexylamin, N-Methylpyrrolidin, N-Methylpiperidin, N-Ethylpiperidin, N,N-Dimethylanilin, N-Methylmorpholin, Pyridin, 2-, 3-, 4-Picolin, 2-Methyl-5-ethyl-pyridin, 2,6-Lutidin, 2,4,6-Collidin, 4-Dimethylaminopyridin, Chinolin, Chinaldin, N,N,N,N-Tetramethylethylendiamin, N,N-Dimethyl-1,4-diazacyclohexan, N,N-Di-ethyl-1,4-diazacyclohexan, 1,8-Bis-(Dimethylamino)naphthalin, Diazabicyclooctan (DABCO), Diazabicyclononan (DBN), Diazabicycloundecan (DBU), Butylimidazol und Methylimidazol.

Beispiele für anorganische Basen sind Alkali- oder Erdalkalimetallhydroxyde, Hydrogencarbonate oder Carbonate und sonstige anorganische wässrige Basen; bevorzugt sind z.B. Natriumhydroxid, Kaliumhydroxid, Lithiumhydroxid, Calciumhydroxid, Natriumcarbonat, Kaliumcarbonat, Natriumhydrogencarbonat, Kaliumhydrogencarbonat und Natriumacetat. Ganz besonders bevorzugt ist Kalium- oder Natriumcarbonat.

Das molare Verhältnis von Basen zum eingesetzten Imid der Formel (II) liegt bevorzugt im Bereich von etwa 0,9 bis etwa 4, besonders bevorzugt im Bereich von etwa 1 bis etwa 3.

Das molare Verhältnis von 2,2-Difluor-1-chlorethan zum eingesetzten Imid der Formel (II) liegt normalerweise im Bereich von etwa 0,3 bis etwa 30, bevorzugt im Bereich von etwa 0,5 bis etwa 10, besonders bevorzugt im Bereich von etwa 1 bis etwa 8, oder von etwa 1 bis etwa 4, oder von etwa 2 bis etwa 4. Das 2,2-Difluor-1-chlorethan kann auch als Lösungsmittel verwendet werden, dann erhöhen sich die vorgenannten Verhältnisse entsprechend.

Das Imid der Formel (II) und die Base können zu dem 2,2-Difluor-1-chlorethan auch zudosiert werden. Die Umsetzung des Schritt (i) wird grundsätzlich unter Eigendruck in einem druckstabilen, geschlossenen Versuchsgefäß (Autoklav) durchgeführt. Der Druck während der Reaktion (d.h. der Eigendruck) ist abhängig von der verwendeten Reaktionstemperatur, von der Menge an 2,2-Difluor-1-chlorethan und von dem verwendeten Lösungsmittel, falls ein Lösungsmittel im Schritt (i) anwesend ist. Ist eine Druckerhöhung gewünscht, so kann eine zusätzliche Druckerhöhung durch Zugabe eines Inertgases, wie Stickstoff oder Argon, erreicht werden.

Das erfindungsgemäße Verfahren kann kontinuierlich oder diskontinuierlich ausgeführt werden. Ebenso ist es denkbar, einige Schritte des erfindungsgemäßen Verfahrens kontinuierlich und die übrigen Schritte diskontinuierlich auszuführen. Kontinuierliche Schritte im Sinne der Erfindung sind solche, bei denen der Zulauf von Verbindungen (Edukte) in einen Reaktor und der Austrag von Verbindungen (Produkte) aus dem Reaktor gleichzeitig aber räumlich getrennt stattfinden, während bei diskontinuierlichen Schritten die Folge Zulauf von Verbindungen (Edukte), ggf. chemische Umsetzung und Austrag von Verbindungen (Produkte) zeitlich nacheinander ablaufen.

Es ist bevorzugt, dass bei Durchführung des Reaktionsschrittes (i) die Innentemperatur im Bereich von etwa 90 °C bis etwa 160 °C, besonders bevorzugt im Bereich von etwa 90 °C bis etwa 140 °C liegt.

Die Reaktionsdauer der Umsetzung in Schritt (i) ist kurz und liegt im Bereich von etwa 0,5 bis etwa 20 Stunden. Eine längere Reaktionsdauer ist möglich, jedoch wirtschaftlich nicht sinnvoll.

Die Aufarbeitung des Reaktionsgemisches aus Schritt (i) ist abhängig von den physikalischen Eigenschaften des Produktes. Wird als Verbindung der Formel (II) Phthalimid oder ein substituiertes Phthalimid verwendet, so wird zuerst das Lösungsmittel im Vakuum entfernt. Wird als Verbindung der Formel (II) Succinimid verwendet, dann werden zuerst die Feststoffe abfiltriert. Daran anschließend erfolgt normalerweise die "Verwässerung" der Reaktionsmischung, d.h. Zugabe von Wasser in dem ggf. Salze gelöst sind. Das Produkt kann dann durch Filtration isoliert bzw. mit Hilfe eines organischen Lösungsmittels aus der wässrigen Phase extrahiert werden.

Im Schritt (ii) erfolgt die Spaltung des Imids der Formel (III) zum 2,2-Difluorethylamin oder einem Salz davon durch das Zugeben von Säure, Base oder Hydrazin. Bevorzugt wird in Schritt (ii) eine Säure oder Hydrazin eingesetzt.

Die in Schritt (ii) verwendbaren Basen sind dem Fachmann bekannt bzw. umfassen die vorliegend als Säurefänger genannten Basen. Die in Schritt (ii) verwendbaren Säuren sind organische oder anorganische Säuren, wobei anorganische Säuren bevorzugt eingesetzt werden. Beispiele solcher erfindungsgemäß bevorzugten anorganischen Säuren sind Salzsäure, Bromwasserstoff, Schwefelsäure und Phosphorsäure.

Die Spaltung des Imids der Formel (III) in Schritt (ii) wird in einem geeigneten Lösungsmittel durchgeführt. Auch hier wird das Lösungsmittel vorzugsweise in einer solchen Menge eingesetzt, dass das Reaktionsgemisch während des gesamten Verfahrens rührbar bleibt. Vorteilhafterweise wird, bezogen auf das eingesetzte Imid der Formel (III), die etwa 1- bis 50-fache Lösungsmittelmenge (v/v), bevorzugt die etwa 2- bis 40-fache Lösungsmittelmenge, besonders bevorzugt die 2- bis 10-fache Lösungsmittelmenge verwendet.

Als Lösungsmittel kommen alle unter den Reaktionsbedingungen inerten organischen Lösungsmittel in Frage. Unter Lösungsmittel werden erfindungsgemäß auch Gemische reiner Lösungsmittel verstanden.

Im Schritt (ii) sind erfindungsgemäß geeignete Lösungsmittel vor allem Wasser, Ether (z.B. Ethylpropylether, Methyl-tert-butylether, *n*-Butylether, Anisol, Phenetol, Cyclohexylmethylether, Dimethylether, Diethylether, Dimethylglycol, Diphenylether, Dipropylether, Diisopropylether, Di-*n-*butylether, Düsobutylether, Diisoamylether, Ethylenglycoldimethylether, Isopropylethylether, Diethylenglykoldimethylether, Triethylenglykoldimethylether, Tetrahydrofuran, 2-Methyltetrahydrofuran, Dioxan, und Polyether des Ethylenoxids und/oder Propylenoxids); aliphatische, cycloaliphatische oder aromatische Kohlenwasserstoffe (z.B. Pentan, Hexan, Heptan, Oktan, Nonan wie die sogenannten "White Spirits" mit Komponenten mit Siedepunkten im Bereich beispielsweise von 40°C bis 250°C, Cymol, Benzinfraktionen innerhalb eines Siedeintervalls von 70°C bis 190°C, Cyclohexan, Methylcyclohexan, Petrolether, Ligroin, Oktan, Benzol, Toluol, Xylol); lineare und verzweigte Carbonsäuren (z.B. Ameisensäure, Essigsäure, Propionsäure, Buttersäure und Isobuttersäure) sowie ihre Ester (z.B. Ethylacetat und Butylacetat), Alkohole (z.B. Methanol, Ethanol, Isopropanol, n-Butanol und Iso-Butanol) oder Gemische davon. Erfindungsgemäß bevorzugte Lösungsmittel im Schritt (ii) sind Methanol, Ethanol und Wasser oder Gemische davon.

Das molare Verhältnis von Säure bzw. Hydrazin zum eingesetzten Imid der Formel (III) liegt im Bereich von etwa 0,8 bis etwa 100, bevorzugt im Bereich von etwa 1 bis etwa 20, besonders bevorzugt im Bereich von etwa 1,1 bis etwa 10. Der Einsatz größerer Mengen an Säure bzw. Hydrazin ist grundsätzlich möglich. Die Säure kann bei geeigneter Handhabbarkeit auch als Lösungsmittel verwendet werden.

Die Spaltung in Schritt (ii) kann bei Temperaturen im Bereich von etwa 0°C bis etwa 150°C durchgeführt werden. Vorzugsweise liegt die Innentemperatur im Bereich von etwa 20°C bis etwa 130°C; besonders bevorzugt liegt sie im Bereich von etwa 40°C bis 110°C.

Die Reaktionsdauer der Spaltung ist kurz und liegt im Bereich von etwa 0,1 bis 12 Stunden. Eine längere Reaktionsdauer ist möglich, jedoch wirtschaftlich nicht sinnvoll.

Nach Reaktionsende kann das erhaltene 2,2-Difluorethylamin durch Destillation aufgereinigt werden. Alternativ kann das 2,2-Difluorethylamin auch als Salz, z.B. Hydrochlorid, isoliert und gereinigt werden. Das 2,2-Difluorethylamin Salz kann anschließend durch Zugabe von Base wieder freigesetzt werden.

Die vorliegende Erfindung wird anhand der nachfolgenden Beispiele näher erläutert, ohne die Erfindung dabei auf diese einzuschränken.

### Herstellungsbeispiele:

### Beispiel 1-Herstellung von 2-(2,2-Difluorethyl)-1H-isoindol-1,3(2H)-dion (Schritt (i))

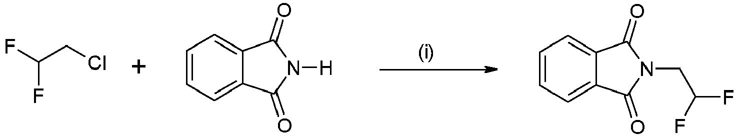

### Beispiel 1.1

27,6 g (0,269 mol) 2,2-Difluor-1-chlorethan, 2,16 g (6,73 mmol) Tetra-n-butylammoniumbromid und 20 g (0,135 mol) Phthalimid werden in 95 g N,N-Dimethylformamid gelöst und mit 46,96 g (0,336 mol) Kaliumcarbonat versetzt. Die Reaktionsmischung wird unter Druck in einem Autoklaven 16 h bei 120°C gerührt. Nach Reaktionsende wird auf Raumtemperatur abgekühlt und das Lösungsmittel weitgehend im Vakuum entfernt. Der verbleibende Rückstand wird mit 150 ml Wasser versetzt und der Feststoff wird abfiltriert. Der Filterrückstand wird zweimal mit Wasser gewaschen und das Produkt wird im Vakuum getrocknet. Man erhält 28,5 g 2-(2,2-Difluorethyl)-1H-isoindol-1,3(2H)-dion mit einer Reinheit von 97,4 %. Dies entspricht einer Ausbeute von 97,7 % der Theorie.

NMR ¹H (CDCl₃): 7,91 (d, 2H), 7,7 (d, 2 H), 6,06 (tt, 1H), 4,07 (dt, 2H).

### Beispiel 1.2

27,6 g (0,269 mol) 2,2-Difluor-1-chlorethan, 2,16 g (6,73 mmol) Tetra-n-butylammoniumbromid und 20 g (0,135 mol) Phthalimid werden in 95 g N,N-Dimethylformamid gelöst und mit 28,18 g (0,201 mol) Kaliumcarbonat versetzt. Die Reaktionsmischung wird unter Druck in einem Autoklaven 16 h bei 120°C gerührt. Nach Reaktionsende wird auf Raumtemperatur abgekühlt und das Lösungsmittel weitgehend im Vakuum entfernt. Der verbleibende Rückstand wird mit 100 ml Wasser versetzt und der Feststoff wird abfiltriert. Der Filterrückstand wird zweimal mit Wasser gewaschen und das Produkt wird im Vakuum getrocknet. Man erhält 24,9 g 2-(2,2-Difluorethyl)-1H-isoindol-1,3(2H)-dion mit einer Reinheit von 99,5 %. Dies entspricht einer Ausbeute von 87,2 % der Theorie.

NMR ¹H (CDCl₃): 7,91 (d, 2H), 7,7 (d, 2 H), 6,06 (tt, 1H), 4,07 (dt, 2H).

### Beispiel 1.3 (Vergleichsbeispiel)

21,4 g (0,214 mol) 2,2-Difluor-1-chlorethan und 20 g (0,107 mol) Kaliumphthalimid werden in 95 g N,N-Dimethylformamid gelöst. Die Reaktionsmischung wird unter Druck in einem Autoklaven 12 h bei 120°C gerührt. Nach Reaktionsende wird auf Raumtemperatur abgekühlt und das Lösungsmittel weitgehend im Vakuum entfernt. Der verbleibende Rückstand wird mit 100 ml Wasser versetzt und der Feststoff wird abfiltriert. Der Filterrückstand wird zweimal mit Wasser gewaschen und das Produkt wird im Vakuum getrocknet. Man erhält 22,5 g 2-(2,2-Difluorethyl)-1H-isoindol-1,3(2H)-dion mit einer Reinheit von nur 59 %. Dies entspricht einer Ausbeute von 59 % der Theorie.

NMR ¹H (CDCl₃): 7,91 (d, 2H), 7,7 (d, 2 H), 6,06 (tt, 1H), 4,07 (dt, 2H).

### Beispiel 1.4

54,25 g (0,529 mol) 2,2-Difluor-1-chlorethan, 9,1 g (0,066 mol) Kaliumcarbonat und 50 g (0,264 mol) Kaliumphthalimid werden in 237 g N,N-Dimethylformamid gelöst. Die Reaktionsmischung wird unter Druck in einem Autoklaven 12 h bei 120°C gerührt. Nach Reaktionsende wird auf Raumtemperatur abgekühlt und das Lösungsmittel weitgehend im Vakuum entfernt. Der verbleibende Rückstand wird mit 250 ml Wasser versetzt und der Feststoff wird abfiltriert. Der Filterrückstand wird zweimal mit Wasser gewaschen und das Produkt wird im Vakuum getrocknet. Man erhält 55,5 g 2-(2,2-Difluorethyl)-1H-isoindol-1,3(2H)-dion mit einer Reinheit von 96,5 %. Dies entspricht einer Ausbeute von 95,8 % der Theorie.

NMR ¹H (CDCl₃): 7,91 (d, 2H), 7,7 (d, 2 H), 6,06 (tt, 1H), 4,07 (dt, 2H).

### Beispiel 1.5

27,6 g (0,269 mol) 2,2-Difluor-1-chlorethan und 20 g (0,135 mol) Phthalimid werden in 95 g N,N-Dimethylformamid gelöst und mit 56,3 g (0,403 mol) Kaliumcarbonat versetzt. Die Reaktionsmischung wird unter Druck in einem Autoklaven 16 h bei 120°C gerührt. Nach Reaktionsende wird auf Raumtemperatur abgekühlt und das Lösungsmittel weitgehend im Vakuum entfernt. Der verbleibende Rückstand wird mit 100 ml Wasser versetzt und der Feststoff wird abfiltriert. Der Filterrückstand wird zweimal mit Wasser gewaschen und das Produkt wird im Vakuum getrocknet. Man erhält 22,2 g 2-(2,2-Difluorethyl)-1H-isoindol-1,3(2H)-dion mit einer Reinheit von 98,4 %. Dies entspricht einer Ausbeute von 77 % der Theorie.

NMR ¹H (CDCl₃): 7,91 (d, 2H), 7,7 (d, 2 H), 6,06 (tt, 1H), 4,07 (dt, 2H).

### Beispiel 2-Herstellung von 1-(2,2-Difluorethvl)pyrrolidin-2,5-dion (Schritt (i))

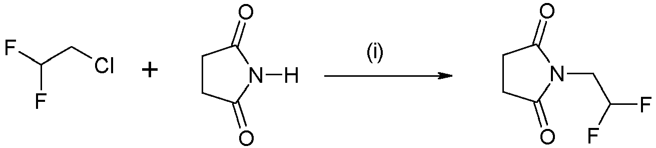

### Beispiel 2.1

204,9 g (1,9 mol) 2,2-Difluor-1-chlorethan, 3,22 g (9,9 mmol) Tetra-n-butylammoniumbromid und 20 g (0,19 mol) Succinimid werden mit 83,6 g (0,599 mol) Kaliumcarbonat versetzt. Die Reaktionsmischung wird unter Druck in einem Autoklaven 16 h bei 120°C gerührt. Nach Reaktionsende wird auf Raumtemperatur abgekühlt und die Reaktionsmischung wird anschließend filtriert. Der Filterrückstand wird mit Dichlormethan gewaschen und das Lösungsmittel wird im Vakuum entfernt. Man erhält 36,5 g 1-(2,2-Difluorethyl)pyrrolidin-2,5-dion mit einer Reinheit von 85 %. Dies entspricht einer Ausbeute von 95 % der Theorie.

NMR ¹H (CDCl₃): 5,99 (tt, 1H), 3,89 (dt, 2H), 2,79(s, 4H),
¹⁹F: -123,15 (dt, CF₂H).

### Beispiel 2.2

40,9 g (0,39 mol) 2,2-Difluor-1-chlorethan, 3,22 g (9,9 mmol) Tetra-n-butylammoniumbromid, 95 g N,N-Dimethylformamid und 20 g (0,19 mol) Succinimid werden mit 83,6 g (0,599 mol) Kaliumcarbonat versetzt. Die Reaktionsmischung wird unter Druck in einem Autoklaven 16 h bei 120°C gerührt. Nach Reaktionsende wird auf Raumtemperatur abgekühlt und die Reaktionsmischung wird anschließend filtriert. Der Filterrückstand wird mit Dichlormethan gewaschen und das Lösungsmittel wird im Vakuum entfernt. Das Öl wird erneut mit 30 ml Wasser versetzt und zweimal mit 30 ml Dichlormethan extrahiert. Die vereinigten organischen Phasen werden getrocknet und das Lösungsmittel im Vakuum entfernt. Man erhält 27,3 g 1-(2,2-Difluorethyl)pyrrolidin-2,5-dion mit einer Reinheit von 93 %. Dies entspricht einer Ausbeute von 77,8 % der Theorie.

NMR ¹H (CDCl₃): 5,99 (tt, 1H), 3,89 (dt, 2H), 2,79(s, 4H).

### Beispiel 3-Herstellung von 2,2-Difluorethylamin (Schritt (ii))

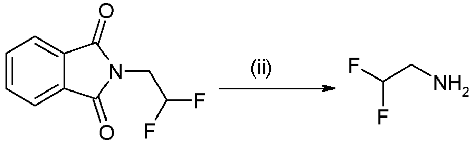

### Beispiel 3.1

In einem Reaktionskolben werden 10 g (0,046 mol) 2-(2,2-Difluorethyl)-1H-isoindol-1,3(2H)-dion in 50 ml Ethanol mit 3,16 g (0,063 mol) Hydrazinhydrat versetzt. Die Reaktionsmischung wird auf Rückfluss erhitzt und 16 h bei Rückfluss gerührt. Es wird auf 50°C abgekühlt und die Reaktionsmischung wird mit 6 ml 32 %iger Salzsäure auf pH 2 gestellt. Es wird nochmals kurz auf Rückfluss erhitzt, anschließend auf Raumtemperatur abgekühlt und der Feststoff abfiltriert. Die Mutterlauge wird zur Trockene eingeengt. Man erhält das 3,9 g 2,2-Difluorethylamin als Hydrochlorid (entspricht 71,4 % der Theorie).

NMR ¹H (D₂O): 6,31 (tt, 1H), 3,51 (dt, 2H).

### Beispiel 3.2

In einem Reaktionskolben werden 10 g (0,046 mol) 2-(2,2-Difluorethyl)-1H-isoindol-1,3(2H)-dion in 50 ml Wasser mit 50 ml 32%ige Salzsäure versetzt. Die Reaktionsmischung wird unter Rückfluss erhitzt und 20 h bei Rückfluss gerührt. Anschließend wird auf Raumtemperatur abgekühlt und der Feststoff abfiltriert. Die Mutterlauge wird zur Trockene eingeengt. Man erhält das 5,2 g 2,2-Difluorethylamin als Hydrochlorid mit einem Gehalt von 93 % (entspricht 88 % der Theorie).

NMR ¹H (D₂O): 6,31 (tt, 1H), 3,51 (dt, 2H).

### Beispiel 4-Herstellung von 2,2-Difluorethylamin (Schritt (ii))

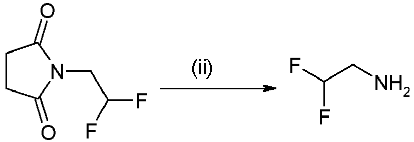

### Beispiel 4.1

In einem Reaktionskolben werden 10 g (0,052 mol) 1-(2,2-Difluorethyl)pyrrolidin-2,5-dion in 50 ml Wasser mit 50 ml 32%iger Salzsäure versetzt. Die Reaktionsmischung wird unter Rückfluss erhitzt und 22 h bei Rückfluss gerührt, anschließend auf Raumtemperatur abgekühlt und der Feststoff abfiltriert. Die Mutterlauge wird zur Trockene eingeengt. Man erhält das 3,1 g 2,2-Difluorethylamin als Hydrochlorid (entspricht 50 % der Theorie).

NMR ¹H (D₂O): 6,31 (tt, 1H), 3,51 (dt, 2H).

## Patentansprüche

1. Ein Verfahren zur Herstellung von 2,2-Difluorethylamin, das folgende Schritte umfasst:
Schritt (i): Umsetzen von 2,2-Difluor-1-chlorethan der Formel (I)
CHF₂-CH₂Cl (I),
mit einem Imid der Formel (II) 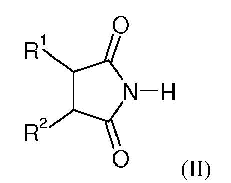 in Gegenwart einer Base, zu einer Verbindung der Formel (III) 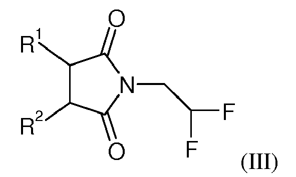 worin in den Verbindungen der Formeln (II) und (III), R¹ und R² jeweils unabhängig voneinander für Wasserstoff oder C₁-C₆-Alkyl stehen, oder R¹ und R² zusammen mit den Kohlenstoffatomen an die sie gebunden sind einen sechsgliedrigen, aromatischen Ring bilden, der gegebenenfalls substituiert ist;
und worin das molare Verhältnis von Base zum eingesetzten Imid der Formel (II) im Bereich von 0,8 bis 5 liegt,
Schritt (ii): Abspaltung von 2,2-Difluorethylamin durch Reaktion der Verbindung der Formel (III) mit Säure, Base oder Hydrazin.

2. Das Verfahren nach Anspruch 1, wobei die Verbindung der Formel (II) als Salz vorliegt.

3. Das Verfahren nach Anspruch 1 oder 2, wobei die Verbindung der Formel (II) Succinimid oder Phthalimid ist.

4. Das Verfahren nach einem der Ansprüche 1 bis 3, wobei die Base in Schritt (i) ausgewählt ist unter tertiären Aminen, substituierten oder unsubstituierten Pyridinen und substituierten oder unsubstituierten Chinolinen, Triethylamin, Trimethylamin, Diisopropylethylamin, Tri-n-propylamin, Tri-n-butylamin, Tri-n-hexylamin, Tricyclohexylamin, N-Methyl-cyclohexylamin, N-Methylpyrrolidin, N-Methylpiperidin, N-Ethylpiperidin, N,N-Dimethylanilin, N-Methylmorpholin, Pyridin, 2-, 3-, 4-Picolin, 2-Methyl-5-ethyl-pyridin, 2,6-Lutidin, 2,4,6-Collidin, 4-Dimethylaminopyridin, Chinolin, Chinaldin, N,N,N,N-Tetramethylenethyldiamin, N,N-Dimethyl-1,4-diazacyclohexan, N,N-Di-ethyl-1,4-diazacyclohexan, 1,8-Bis-(Dimethyl-amino)naphthalin, Diazabicyclooctan (DABCO), Diazabicyclononan (DBN), Diazabicycloundecan (DBU), Butylimidazol, Methylimidazol, Natriumhydroxid, Kaliumhydroxid, Lithiumhydroxid, Calciumhydroxid, Natriumcarbonat, Kaliumcarbonat, Natriumhydrogencarbonat, Kaliumhydrogencarbonat und Natriumacetat.

5. Das Verfahren nach einem der Ansprüche 1 bis 3, wobei die Base in Schritt (i) Kaliumcarbonat oder Natriumcarbonat ist.

6. Das Verfahren nach einem der Ansprüche 1 bis 5 wobei in Schritt (i) weiterhin ein Katalysator anwesend ist, wobei der Katalysator ausgewählt ist unter Alkalibromiden und -jodiden, Ammoniumbromid, Ammoniumjodid, Tetraalkylammoniumbromiden und -jodiden, Tetraalkyl- oder Tetraarylphosphoniumhalogeniden, Tetrahis(dimethylamino)phosphoniumbromid, Tetrahis(diethylamino)phosphoniumbromid, Tetrakis(dipropylamino)phosphoniumchlorid bzw. -bromid und Bis(dimethylamino)[(1,3-dimethylimidazolidin-2-yliden)amino] methyliumbromid.

7. Das Verfahren nach Anspruch 6, wobei der Katalysator Natriumbromid, Kaliumbromid, Natriumjodid, Kaliumjodid oder Tetrabutylammoniumbromid ist.

8. Das Verfahren nach einem der Ansprüche 1 bis 7, wobei in Schritt (ii) anorganische Säuren eingesetzt werden.

9. Das Verfahren nach Anspruch 8, wobei die anorganische Säure Salzsäure, Bromwasserstoff, Schwefelsäure oder Phosphorsäure ist.

10. Das Verfahren nach einem der Ansprüche 1 bis 9, wobei das molare Verhältnis von Säure zum Imid der Formel (III) im Bereich von 0,8 bis 100 liegt.

## Claims

1. Process for the preparation of 2,2-difluoroethylamine comprising the following stages:
Stage (i): Reaction of 2,2-difluoro-1-chloroethane of the formula (I)
CHF₂-CH₂Cl (I)
with an imide of the formula (II) 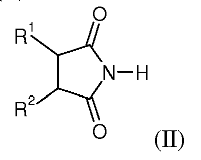 in the presence of a base, to give a compound of the formula (III) 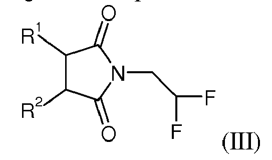 in which, in the compounds of the formulae (II) and (III), R¹ and R² are, each independently of one another, hydrogen or C₁-C₆-alkyl or R¹ and R² form, together with the carbon atoms to which they are bonded, a six-membered aromatic ring which is optionally substituted;
and in which the molar ratio of base to the imide of the formula (II) used lies in the range from 0.8 to 5,
Stage (ii): Cleavage of 2,2-difluoroethylamine by reaction of the compound of the formula (III) with acid, base or hydrazine.

2. Process according to Claim 1, in which the compound of the formula (II) is present as salt.

3. Process according to Claim 1 or 2, in which the compound of the formula (II) is succinimide or phthalimide.

4. Process according to one of Claims 1 to 3, in which the base in stage (i) is chosen from tertiary amines, substituted or unsubstituted pyridines and substituted or unsubstituted quinolines, triethylamine, trimethylamine, diisopropylethylamine, tri-n-propylamine, tri-n-butylamine, tri-n-hexylamine, tricyclohexylamine, N-methylcyclohexylamine, N-methylpyrrolidine, N-methylpiperidine, N-ethylpiperidine, N,N-dimethylaniline, N-methylmorpholine, pyridine, 2-, 3- or 4-picoline, 2-methyl-5-ethylpyridine, 2,6-lutidine, 2,4,6-collidine, 4-dimethylaminopyridine, quinoline, quinaldine, N,N,N,N-tetramethylethylenediamine, N,N-dimethyl-1,4-diazacyclohexane, N,N-diethyl-1,4-diazacyclohexane, 1,8-bis(dimethylamino)naphthalene, diazabicyclooctane (DABCO), diazabicyclononane (DBN), diazabicycloundecane (DBU), butylimidazole, methylimidazole, sodium hydroxide, potassium hydroxide, lithium hydroxide, calcium hydroxide, sodium carbonate, potassium carbonate, sodium hydrogencarbonate, potassium hydrogencarbonate and sodium acetate.

5. Process according to one of Claims 1 to 3, in which the base in stage (i) is potassium carbonate or sodium carbonate.

6. Process according to one of Claims 1 to 5, in which a catalyst is furthermore present in stage (i), the catalyst being chosen from alkali metal bromides and iodides, ammonium bromide, ammonium iodide, tetraalkylammonium bromides and iodides, tetraalkyl- or tetraarylphosphonium halides, tetrakis(dimethylamino)phosphonium bromide, tetrakis(diethylamino)phosphonium bromide, tetrakis(dipropylamino)phosphonium chloride, tetrakis(dipropylamino)phosphonium bromide and bis(dimethylamino)[(1,3-dimethylimidazolidin-2-ylidene)amino]methylium bromide.

7. Process according to Claim 6, in which the catalyst is sodium bromide, potassium bromide, sodium iodide, potassium iodide or tetrabutylammonium bromide.

8. Process according to one of Claims 1 to 7, in which inorganic acids are used in stage (ii).

9. Process according to Claim 8, in which the inorganic acid is hydrochloric acid, hydrobromic acid, sulphuric acid or phosphoric acid.

10. Process according to one of Claims 1 to 9, in which the molar ratio of acid to the imide of the formula (III) lies in the range from 0.08 to 100.

## Revendications

1. Procédé pour la préparation de 2,2-difluoroéthylamine, comprenant les étapes suivantes :
étape (i) _{:} transformation de 2,2-difluoro-1-chloroéthane de formule (I)
CHF₂-CH₂Cl (I),
avec un imide de formule (II) 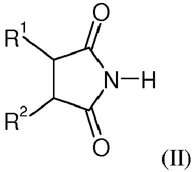 en présence d'une base, en un composé de formule (III) 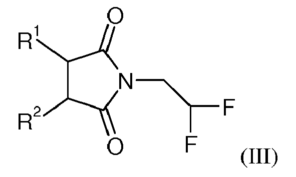 où, dans les composés des formules (II) et (III), R¹ et R² représentent à chaque fois, indépendamment, hydrogène ou C₁-C₆-alkyle ou R¹ et R² forment ensemble avec les atomes de carbone auxquels ils sont liés, un cycle aromatique à six chaînons, qui est le cas échéant substitué ;
et où le rapport molaire de la base à l'imide utilisé de formule (II) se situe dans la plage de 0,8 à 5,
étape (ii) : dissociation de 2,2-difluoroéthylamine par réaction du composé de formule (III) avec un acide, une base ou de l'hydrazine.

2. Procédé selon la revendication 1, le composé de formule (II) se trouvant sous forme de sel.

3. Procédé selon la revendication 1 ou 2, le composé de formule (II) étant un succinimide ou un phtalimide.

4. Procédé selon l'une quelconque des revendications 1 à 3, la base dans l'étape (i) étant choisie parmi les amines tertiaires, les pyridines substituées ou non substituées et les quinoléines substituées ou non substituées, la triéthylamine, la triméthylamine, la diisopropyléthylamine, la tri-n-propylamine, la tri-n-butylamine, la tri-n-hexylamine, la tricyclohexylamine, la N-méthylcyclohexylamine, la N-méthylpyrrolidine, la N-méthylpipéridine, la N-éthylpipéridine, la N,N-diméthylaniline, la N-méthylmorpholine, la pyridine, la 2-picoline, la 3-picoline, la 4-picoline, la 2-méthyl-5-éthylpyridine, la 2,6-lutidine, la 2,4,6-collidine, la 4-diméthylaminopyridine, la quinoléine, la quinaldine, la N,N,N,N-tétraméthyléthylènediamine, le N,N-diméthyl-1,4-diazacyclohexane, le N,N-diéthyl-1,4-diazacyclohexane, le 1,8-bis-(diméthylamino)naphtalène, le diazabicyclooctane (DABCO), le diazabicyclononane (DBN), le diazabicyclo-undécane (DBU), le butylimidazole, le méthylimidazole, l'hydroxyde de sodium, l'hydroxyde de potassium, l'hydroxyde de lithium, l'hydroxyde de calcium, le carbonate de sodium, le carbonate de potassium, l'hydrogénocarbonate de sodium, l'hydrogénocarbonate de potassium et l'acétate de sodium.

5. Procédé selon l'une quelconque des revendications 1 à 3, la base dans l'étape (i) étant le carbonate de potassium ou le carbonate de sodium.

6. Procédé selon l'une quelconque des revendications 1 à 5, un catalyseur étant en outre présent dans l'étape (i), le catalyseur étant choisi parmi les bromures et les iodures de métal alcalin, le bromure d'ammonium, l'iodure d'ammonium, les bromures et les iodures de tétraalkylammonium, les halogénures de tétraalkylphosphonium ou de tétraarylphosphonium, le bromure de tétrakis(diméthylamino)phosphonium, le bromure de tétrakis(diéthylamino)phosphonium, le chlorure ou le bromure de tétrakis(dipropylamino)phosphonium et le bromure de bis(diméthylamino)[(1,3-diméthylimidazolidin-2-ylidène)amino]méthylium.

7. Procédé selon la revendication 6, le catalyseur étant le bromure de sodium, le bromure de potassium, l'iodure de sodium, l'iodure de potassium ou le bromure de tétrabutylammonium.

8. Procédé selon l'une quelconque des revendications 1 à 7, des acides inorganiques étant utilisés dans l'étape (ii).

9. Procédé selon la revendication 8, l'acide inorganique étant l'acide chlorhydrique, l'acide bromhydrique, l'acide sulfurique ou l'acide phosphorique.

10. Procédé selon l'une quelconque des revendications 1 à 9, le rapport molaire d'acide à imide de formule (III) se situant dans la plage de 0,8 à 100.
